(19) Europäisches Patentamt
European Patent Office
Office européen des brevets

(11) EP 0 296 453 B1

(12) # EUROPÄISCHE PATENTSCHRIFT

(45) Veröffentlichungstag und Bekanntmachung des Hinweises auf die Patenterteilung:
**13.08.1997 Patentblatt 1997/33**

(21) Anmeldenummer: **88109426.2**

(22) Anmeldetag: **14.06.1988**

(51) Int Cl.6: **C07D 487/04**, C07D 471/04, C07D 513/04, A01N 43/90 // (C07D487/04, 239:00, 235:00), (C07D471/04, 235:00, 221:00), (C07D513/04, 275:00, 235:00), (C07D487/04, 235:00, 209:00), (C07D487/04, 239:00, 239:00)

(54) **Nitro-substituierte heterocyclische Verbindungen**

Nitrosubstituted heterocyclic compounds

Composés hétérocycliques substitués par un groupe nitro

(84) Benannte Vertragsstaaten:
**BE CH DE FR GB IT LI NL**

(30) Priorität: **26.06.1987 JP 157528/87**

(43) Veröffentlichungstag der Anmeldung:
**28.12.1988 Patentblatt 1988/52**

(73) Patentinhaber: **NIHON BAYER AGROCHEM K.K.**
**Tokyo 108 (JP)**

(72) Erfinder:
- **Shiokawa, Kozo**
  **Kawasaki-shi Kanagwa-ken (JP)**
- **Tsuboi, Shinichi**
  **Hino-shi Tokyo (JP)**
- **Sasaki, Shoko**
  **Hino-shi Tokyo (JP)**
- **Moriya, Koichi**
  **Tokyo (JP)**
- **Hattori, Yumi**
  **Hachioji-shi Tokyo (JP)**
- **Shibuya, Katsuhiko**
  **Hachioji-shi Tokyo (JP)**

(74) Vertreter: **Schumacher, Günter, Dr. et al**
**Bayer AG**
**Konzernbereich RP**
**Patente und Lizenzen**
**51368 Leverkusen (DE)**

(56) Entgegenhaltungen:
**EP-A- 0 247 177** **US-A- 4 031 087**

- **CHEMISCHE BERICHTE, Band 119, Heft 5, 1986, VCH Verlags- gesellschaft, Weinheim ZHI-TANG HUANG et al. "Synthesis of ketene aminals with an imidazolidine ring by condensation 4,5-dihydro-2- (methylthio)-1H-imidazoles with active methylene compounds and some addition and cyclocondensation reactions." Seiten 2208-2219**
- **HETEROCYCLES, Band 15, Nr. 1, 1981 EUGEN UHLMANN et al. "Pteri- dines, LXX- synthesis and properties of 1,8-alkylene- bridged lumazines." Seiten 437-453**
- **JOURNAL OF THE CHEMICAL SOCIETY, Perkin Transaction I,Band 7, 1979 NARAYANA IYER VISWANATHAN et al. "4-Nitro-1,2,3-triazoles from Nitro-alphabeta-un- saturated gem-diamines." Seiten 2361-2363**
- **JOURNAL OF HETEROCYCLIC CHEMISTRY, Band 17, Nr. 5, Juli 1980 KURT PILGRAM "Synthesis of 2,3-dihydro-1H-imidazo/1,2-b/-pyrazoles." Seiten 1413-1416**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 296 453 B1

## Beschreibung

Die vorliegende Erfindung betrifft neue nitro-substituierte heterocyclische Verbindungen, Verfahren zu ihrer Herstellung und ihre Verwendung als Insektizide.

Die folgenden mit den beanspruchten Verbindungen strukturell verwandten nitro-substituierten heterocyclischen Verbindungen der Formeln (A) bis (E) sind bereits in der Literatur offenbart:

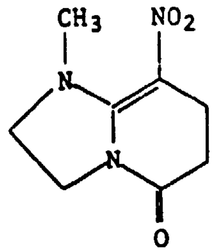

(A)

(siehe Chemische Berichte, 1968, Band 119, Seiten 2208-2219)

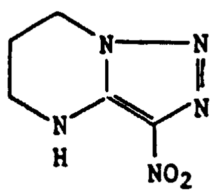

(B)

(siehe J. Chem. Soc., Perkin Transactions I, 1979, Seiten 2361-2363)

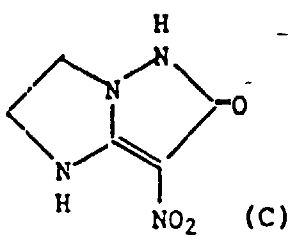

(C)

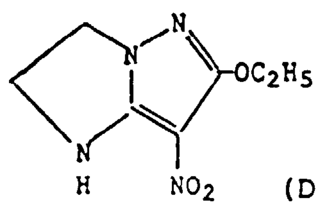

(D)

(siehe J. Heterocycl. Chem. 1980, Band 17, Seite 1413)

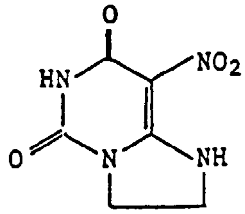

(E)

(siehe Heterocycles, 1980, Band 15, Seite 437)

Nunmehr wurden neue nitro-substituierte heterocyclische Verbindungen der Formel (I)

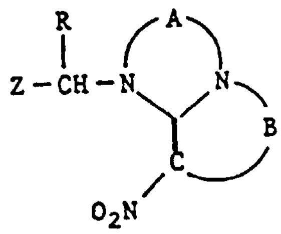

(I)

gefunden, in der

- R Wasserstoff oder $C_1$-$C_4$-Alkyl ist,
- Z $C_6$-$C_{10}$-Aryl oder eine 5- bis 6-gliedrige heterocyclische Gruppe, die 1 bis 2 aus O, S und N ausgewählte Hetero-Atom enthält, von denen wenigstens eines ein Stickstoff-Atom ist, ist, wobei die Aryl-Gruppe oder die heterocyclische Gruppe jeweils durch einen Substituenten substituiert sein kann, der beliebig aus der aus Halogen, $C_1$-$C_4$-Alkyl, $C_1$-$C_4$-Alkoxy, $C_1$-$C_4$-Halogenoalkyl, $C_1$-$C_4$-Alkylthio, $C_1$-$C_4$-Halogenoalkoxy, $C_1$-$C_4$-Halogenoalkylthio, Nitro und Cyano bestehenden Gruppe ausgewählt ist,
- A Ethylen oder Trimethylen ist, die beide durch Methyl substituiert sein können, und
- B für 2 Glieder eines heterocyclischen Ringes steht, der zusammen mit dem benachbarten C-Atom und dem benachbarten N-Atom gebildet wird und worin wenigstens eines der Glieder, die gegebenenfalls substituiert sein können, ein Stickstoff-Atom oder ein Schwefel-Atom bezeichen kann,

wobei die Glieder B gegebenenfalls durch wenigstens einen Substituenten substituiert sein können, der aus der aus Halogen, $C_1$-$C_6$-Alkyl, $C_1$-$C_4$-Alkoxy, Alkoxycarbonyl mit 1 bis 4 Kohlenstoff-Atomen in dem Alkoxy-Teil, $C_6$-$C_{10}$-Aryl, Keto, Imino, Phenoxy, $C_1$-$C_4$-Alkoxythio, Alkoxycarbonylimino mit 1 bis 4 Kohlenstoff-Atomen in dem Alkoxy-Teil, Phenoxycarbonylimino, Benzoylimino, Benzyl, Cyano, Thioketo, Hydroxy und $C_1$-$C_2$-Alkyliden bestehenden Gruppe ausgewählt ist.

Hervorgehoben sind nitrosubstituierte heterocyclische Verbindungen der Formel

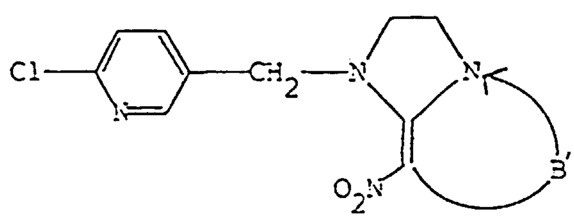

worin
B' für eine der folgenden Gruppierungen steht:

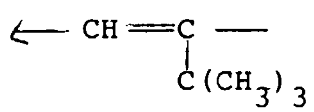

,

$\leftarrow CH{=}CH—$ ,

Es wurden Verfahren zur Herstellung von nitro-substituierten heterocyclischen Verbindungen der Formel (I)

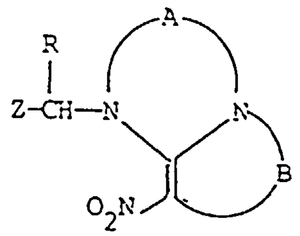

(I)

in der R, Z, A und B die oben angegebene Bedeutung haben, gefunden,die dadurch gekennzeichnet sind, daß Verbindungen der Formel (II)

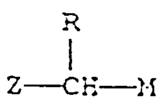

(II),

in der

R und Z jeweils die im Vorstehenden angegebenen Bedeutungen haben,
M Halogen oder $-OSO_2-L$, worin L Alkyl oder Aryl bezeichnet, das substituiert sein kann, darstellt,

mit Verbindungen der Formel (III)

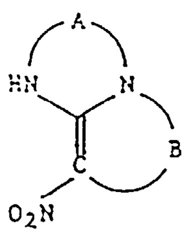

(III)

,

in der A und B die im Vorstehenden angegebenen Bedeutungen haben, in Gegenwart inerter Lösungsmittel und gegebenenfalls in Gegenwart einer Base umgesetzt werden.

Die nitro-substituierten heterocyclischen Verbindungen der Formel (I) gemäß der vorliegenden Erfindung zeigen starke insektizide Eigenschaften.

Darüber hinaus ist es überraschend, daß die erfindungsgemäßen nitro-substituierten heterocyclischen Verbindungen der Formel (I) im Vergleich zu den bekannten Verbindungen, die Strukturen analog zu denen der vorliegenden erfindungsgemäßen Verbindungen haben, beträchtlich verbesserte insektizide Eigenschaften aufweisen.

Ganz besonders bevorzugte nitro-substituierte heterocyclische Verbindungen der Formel (I) sind diejenigen, in denen

R Wasserstoff oder Methyl ist,
Z Phenyl oder eine 5- bis 6-gliedrige heterocyclische Gruppe, die 1 bis 2 aus O, S und N ausgewählte Hetero-Atom enthält, von denen wenigstens eines ein Stickstoff-Atom ist, ist, wobei die Phenyl-Gruppe oder die heterocyclische Gruppe jeweils durch einen oder zwei Substituenten substituiert sein kann, der beliebig aus der aus Fluor, Chlor, Brom, Methyl, Ethyl, Methoxy, Trifluoromethyl, Methylthio, Trifluoromethoxy, Trifluoromethylthio, Nitro und Cyano bestehenden Gruppe ausgewählt ist,
A Ethylen oder Trimethylen ist, die beide durch Methyl substituiert sein können, und
B für 2 Glieder eines heterocyclischen Ringes steht, der zusammen mit dem benachbarten C-Atom und dem benachbarten N-Atom gebildet wird, worin wenigstens eines der Glieder ein Stickstoff-Atom oder ein Schwefel-Atom bezeichnen kann, wobei die Glieder gegebenenfalls durch wenigstens einen-Substituenten substituiert sein können,

der aus der aus Fluor, Chlor, Brom, Methyl, Methoxy, Alkoxycarbonyl mit 1 bis 2 Kohlenstoff-Atomen in dem Alkoxy-Teil, Phenyl, Keto, Imino, Phenoxy, Alkoxycarbonylimino mit 1 bis 2 Kohlenstoff-Atomen in dem Alkoxy-Teil, Phenoxycarbonylimino, Benzoylimino, Benzyl, Cyano, Thioketo, Hydroxy und $C_1$-$C_2$-Alkyliden bestehenden Gruppe ausgewählt ist.

Als Einzelbeispiele für die erfindungsgemäßen Verbindungen der Formel (I) können die folgenden Verbindungen aufgeführt werden:

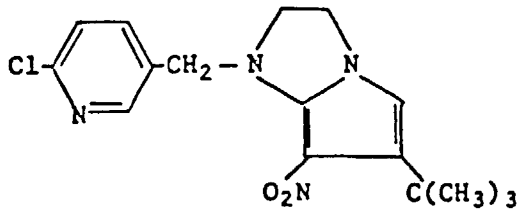

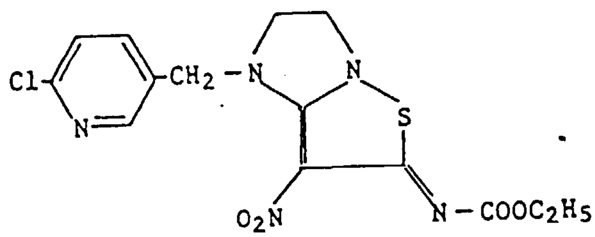

,

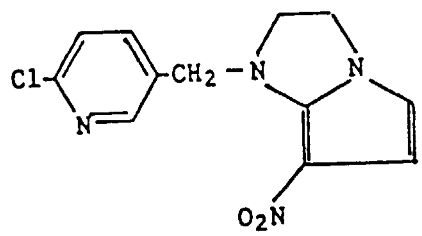

.

Wenn in dem oben bezeichneten Verfahren beispielsweise als Ausgangs-Verbindungen 2-Chloro-5-chloromethylpyridin und Ethyl-1,2,3,5-tetrahydro-8-nitro-imidazo-[1,2-a]pyridin-5-on-6-carboxylat eingesetzt werden, kann die Reaktion wie folgt dargestellt werden:

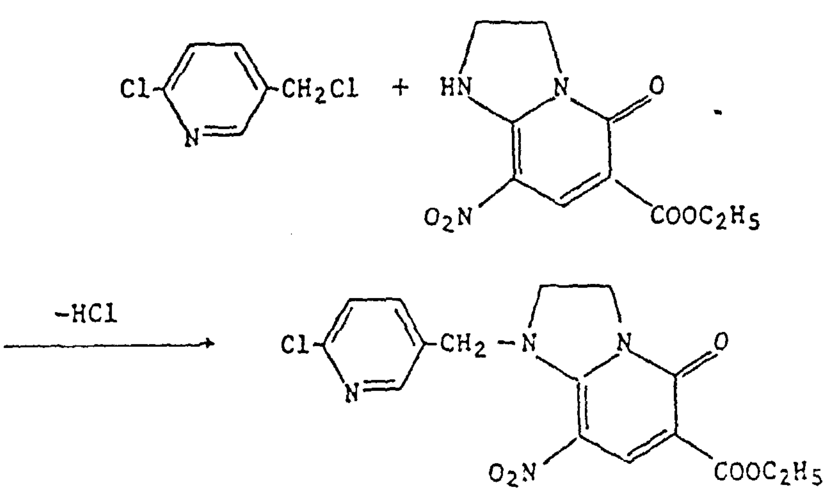

In den Verbindungen der Formel (II) im oben bezeichneten Verfahren a) haben R, Z und M die im Vorstehenden angegebenen Bedeutungen.

In der Formel (II) haben R und Z vorzugsweise jeweils die im Vorstehenden als bevorzugt angegebenen Bedeutungen, und M ist vorzugsweise Chlor oder Tosyloxy.

Die Verbindungen der Formel (II) sind in der Fachwelt bereits bekannt, beispielsweise aus der JP-OS 81382/1987, und als Einzelbeispiele seien 2-Chloro-5-chloromethylpyridin und 2-Chloro-5-chloromethylthiazol genannt.

In den Verbindungen der Formel (III) im Verfahren a) haben A und B die im Vorstehenden angegebenen Bedeutungen.

In der Formel (III) haben A und B vorzugsweise jeweils die im Vorstehenden als bevorzugt angegebenen Bedeutungen.

Die Verbindungen der Formel (III) umfassen bekannte Verbindungen, die beispielsweise in Journal of the Chemical Society, Perkin Transactions I, 1979, Seite 2361, Journal of Heterocyclic Chemistry Band 17, Seite 1413 und Chemische Berichte, Band 119, Seite 2208, beschrieben sind.

Ein Teil der Verbindungen der Formel (III) kann dadurch erhalten werden, daß die durch die nachstehende Formel (XVII)

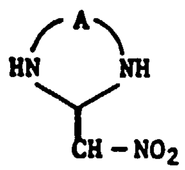

(XVII)

,

in der A die im Vorstehenden angegebenen Bedeutungen hat, bezeichneten Verbindungen entsprechend dem Verfahren b) mit den Verbindungen der im Vorstehenden bezeichneten Formeln (V), (VI), (VII), (VIII) , (IX), (XI), umgesetzt werden.

Die Verbindungen der obigen Formel (XVII) sind bereits bekannt, beispielsweise aus der JP-OS 218 386/1985, und als Einzelbeispiele seien 2-Nitromethylen-imidazolidin und 2-Nitromethylen-tetrahydropyrimidin genannt.

Darüber hinaus sind die Verbindungen der Formeln (V), (VI), (VII), (VIII), (IX), (XI), auf dem Gebiet der organischen Chemie wohlbekannt.

Weiterhin können beispielsweise die durch die nachstehende Formel (XVIII), die der Formel (III) entspricht,

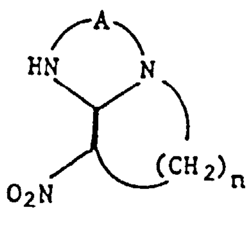

(XVIII)

,

in der A die im Vorstehenden angegebene Bedeutung hat und n 2 ist, bezeichneten Verbindungen durch Zersetzung der Verbindungen der nachstehenden Formel (XIX)

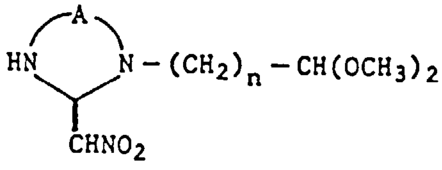

(XIX)

,

in der A und n die im Vorstehenden angegebenen Bedeutungen haben, mit Hilfe einer Säure und nachfolgende Cyclisierung des Produkts erhalten werden.

Die Verbindungen der Formel (XIX) können durch Umsetzung einer Verbindung der Formel (XX)

$$H_2N—A—NH—(CH_2)_n—CH(OCH_3)_2 \qquad (XX),$$

in der A und n die im Vorstehenden angegebenen Bedeutungen haben, mit 1-Nitro-2,2-bis(methylthio)ethylen erhalten werden.

Die Verbindungen der Formel (XX) können durch Umsetzung der Verbindungen der nachstehenden Formel (XXI)

$$H_2N—A—NH_2 \qquad (XXI),$$

in der A die im Vorstehenden angegebenen Bedeutungen hat, mit einer Verbindung der nachstehenden Formel (XXII)

$$Hal—(CH_2)_n—CH(OCH_3)_2 \qquad (XXII),$$

in der n die im Vorstehenden angegebenen Bedeutungen hat und Hal ein Halogen-Atom ist, erhalten werden.

Sowohl die Verbindungen der vorstehenden Formel (XXI) als auch die Verbindungen der vorstehenden Formel (XXII) sind bekannte Verbindungen.

In den Verbindungen der Formel (IV) im Verfahren b) haben R, Z und A jeweils die im Vorstehenden angegebenen Bedeutungen.

In der Formel (IV) haben R, Z und A jeweils vorzugsweise die im Vorstehenden als bevorzugt angegebenen Bedeutungen.

Die Verbindungen der obigen Formel (IV) sind bereits bekannt, beispielsweise aus der JP-OS 218 386/1985 und der JP-OS 183 271/1986, und als konkrete Beispiele seien genannt:

1-(2-Chloro-5-pyridylmethyl)-2-nitromethylenimidazolidin,
1-(2-Chloro-5-pyridylmethyl)-2-nitromethylentetrahydropyrimidin,
1-(2-Chloro-5-thiazolylmethyl)-2-nitromethylenimidazolidin,
1-(2-Fluoro-5-pyridylmethyl)-2-nitromethylenimidazolidin,
1-(2-Methyl-5-pyridylmethyl)-2-nitromethylenimidazolidin.

Bei der Durchführung des oben bezeichneten Verfahrens a) können als geeignete Verdünnungsmittel beliebige inerte Verdünnungsmittel eingesetzt werden.

Beispiele für die Lösungsmittel oder Verdünnungsmittel sind Wasser; aliphatische, cycloaliphatische und aromatische, gegebenenfalls chlorierte, Kohlenwasserstoffe, etwa Hexan, Cyclohexan, Petrolether, Ligroin, Benzol, Toluol, Xylol, Methylenchlorid, Chloroform, Kohlenstofftetrachlorid, Ethylenchlorid, Trichloroethylen, Chlorbenzol und dergleichen; Ether, etwa Diethylether, Methylethylether, Diisopropylether, Dibutylether, Propylenoxid, Dioxan, Tetrahydrofuran und dergleichen; Nitrile, etwa Acetonitril, Propionitril, Acrylnitril und dergleichen; Alkohole, etwa Methanol, Ethanol, Isopropanol, Butanol, Ethylenglycol und dergleichen; Säureamide, etwa Dimethylformamid, Dimethylacetamid und dergleichen; und Sulfone und Sulfoxide, etwa Dimethylsulfoxid, Sulfolan und dergleichen; sowie Basen, beispielsweise Hydride wie Natriumhydrid und Kaliumhydrid, Alkalimetallhydroxide, -carbonate und tertiäre Amine wie Triethylamin.

Bei dem oben bezeichneten Verfahren a) kann die Reaktionstemperatur innerhalb eines breiten Bereichs variiert werden. Im allgemeinen wird die Reaktion bei einer Temperatur von etwa 0 °C bis 100 °C, vorzugsweise von etwa 10 °C bis 80 °C, durchgeführt.

Im allgemeinen läßt man die Reaktion unter Normaldruck ablaufen, obwohl es auch möglich ist, einen höheren oder niedrigeren Druck anzuwenden.

Wenn das oben bezeichnete Verfahren a) gemäß der vorliegenden Erfindung durchgeführt wird, werden beispielsweise etwa 1,1 bis 1,2 mol Natriumhydrid als Base und etwa 1 bis 1,2 mol, vorzugsweise etwa 1 bis 1,1 mol, der Verbindungen (II) pro 1 mol der Verbindungen (III) eingesetzt. Die Reaktion kann in Gegenwart eines inerten Lösungsmittels wie, beispielsweise, Dimethylsulfoxid durchgeführt werden, um die angestrebte Verbindung zu erhalten.

Wenn das oben bezeichnete Verfahren b) durchgeführt wird, ist es möglich, irgendein Lösungsmittel einzusetzen, das dem im Verfahren a) gemäß der vorliegenden Erfindung entspricht.

Das oben bezeichnete Verfahren b) kann bei einer Reaktionstemperatur durchgeführt werden, die innerhalb eines breiten Bereichs variiert, beispielsweise zwischen etwa 0 °C und dem Siedepunkt der Mischung, vorzugsweise zwischen etwa 0 °C und etwa 100 °C.

Die Reaktion wird vorzugsweise unter Normaldruck durchgeführt, obwohl es auch möglich ist, einen höheren oder niedrigeren Druck anzuwenden.

In dem Verfahren b) kann die Reaktion der Verbindungen der Formel (IV) entweder mit der Verbindung der Formel (VI) oder mit den Verbindungen der Formel (VII) in ähnlicher Weise wie die Reaktionen durchgeführt werden, die in den US-Patentschriften 4 033 954 und 4 033 955 beschrieben sind.

Weiterhin kann die Reaktion der Verbindungen der Formel (IV) mit der Verbindung der Formel (IX) in Analogie zu einer Reaktion durchgeführt werden, die im Journal für praktische Chemie, Band 319, Seite 149, beschrieben ist.

In dem Fall, in dem die Formel (I) der vorliegenden Erfindung durch die nachstehende Formel (Ib)

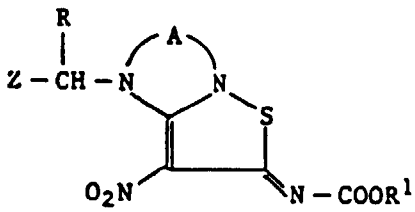

(Ib)

repräsentiert wird, in der R, Z, A und $R^1$ die im Vorstehenden angegebenen Bedeutungen haben, können die betreffenden Verbindungen unabhängig von den oben angegebenen Verfahren a) und b) dadurch erhalten werden, daß die Verbindungen der Formel (IV) mit den Verbindungen der nachstehenden Formel (XXIII)

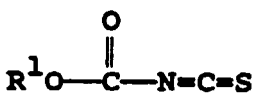

(XXIII),

in der $R^1$ die im Vorstehenden angegebene Bedeutungen hat, umgesetzt werden und danach das Produkt mit Brom umgesetzt wird.

Die vorstehende Reaktion kann nach den in den Veröffentlichungen in Tetrahedron Band 33, S. 1057, und ebenda, Band 37, Seite 1470, und in Indian Journal of Chemistry 15B, S. 886, beschriebenen Verfahren durchgeführt werden.

Weiterhin können durch Hydrolyse der Verbindungen der vorstehenden Formel (Ib) unter milden Bedingungen die der Formel (I) der vorliegenden Erfindung entsprechenden Verbindungen der nachstehenden Formel (Ic)

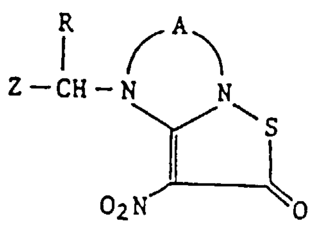

(Ic)

in der R, Z und A die im Vorstehenden angegebenen Bedeutungen haben, erhalten werden.

Die erfindungsgemäßen Wirkstoffe werden von Pflanzen gut vertragen, haben einen günstigen Wert der Toxizität gegenüber warmblütigen Tieren und lassen sich einsetzen zur Bekämpfung von Arthropoden-Schädlingen, insbesondere von Insekten, die in der Land- und Forstwirtschaft auftreten, zum Schutz von Lagerprodukten und Materialien und auf dem Gebiet der Hygiene. Sie sind gegenüber normal empfindlichen und resistenten Species sowie gegen alle oder einige Entwicklungsstadien aktiv. Zu den oben genannten Schädlingen zählen:
Aus der Klasse der Isopoda beispielsweise

Oniscus asellus,
Armadillidium vulgare und
Porcellio scaber;

aus der Klasse der Diplopoda beispielsweise
Blaniulus guttulatus;
aus der Klasse der Chilopoda beispielsweise

Geophilus carpophagus und
Scutigera spec.;

aus der Klasse der Symphyla beispielsweise
Scutigerella immaculata;
aus der Ordnung der Thysanura beispielsweise
Lepisma saccharina;
aus der Ordnung der Collembola beispielsweise
Onychiurus armatus; aus der Ordnung der Orthoptera beispielsweise

Blatta orientalis,
Periplaneta americana,
Leucophaea maderae,
Blatella germanica,
Acheta domesticus,
Gryllotalpa spp.,
Locusta migratoria migratorioides,
Melanoplus differentialis und
Schistocerca gregaria;

aus der Ordnung der Dermaptera beispielsweise
Forficula auricularia,
aus der Ordnung der Isoptera beispielsweise
Reticulitermes spp.;
aus der Ordnung der Anoplura beispielsweise

Phylloxera vastatrix,
Pemphigus spp.,
Pediculus humanus corporis,
Haematopinus spp. und
Linognathus spp.;

aus der Ordnung der Mallophaga beispielsweise

Trichodectes spp. und
Damalinea spp.;

aus der Ordnung der Thysanoptera beispielsweise

Hercinothrips femoralis und
Thrips tabaci;

aus der Ordnung der Heteroptera beispielsweise

Eurygaster spp.,
Dysdercus intermedius,
Piesma quadrata,
Cimex lectularius,
Rhodnius prolixus und
Triatoma spp.;

aus der Ordnung der Homoptera beispielsweise

Aleurodes brassicae,
Bemisia tabaci,
Trialeurodes vaporariorum,
Aphis gossypii,
Brevicoryne brassicae,

Cryptomyzus ribis,
Aphis fabae,
Doralis pomi,
Eriosoma lanigerum,
Hyalopterus arundinis,
Macrosiphum avenae,
Myzus spp.,
Phorodon humuli,
Rhopalosiphum padi,
Empoasca spp.,
Euscelis bilobatus,
Nephotettix cincticeps,
Lecanium corni,
Saissetia oleae,
Laodelphax striatellus,
Nilaparvata lugens,
Aonidiella aurantii,
Aspidiotus hederae,
Pseudococcus spp. und
Psylla spp.;

aus der Ordnung der Lepidoptera beispielsweise

Pectinophora gossypiella,
Bupalus piniarius,
Cheimatobia brumata,
Lithocolletis blancardella,
Hyponomeuta padella,
Plutella maculipennis,
Malacosoma neustria,
Euproctis chrysorrhoea,
Lymantria spp.,
Bucculatrix thurberiella,
Phyllocnistis citrella,
Agrotis spp.,
Euxoa spp.,
Feltia spp.,
Earias insulana,
Heliothis spp.,
Spodoptera exigua,
Mamestra brassicae,
Panolis flammea,
Prodenia litura,
Spodoptera spp.,
Trichoplusia ni,
Carpocapsa pomonella,
Pieris spp.,
Chilo spp.,
Pyrausta nubilalis,
Ephestia kuehniella,
Galleria mellonella,
Cacoecia podana,
Capua reticulana,
Choristoneura fumiferana,
Clysia ambiguella,
Homona magnanima und
Tortrix viridana;

aus der Ordnung der Coleoptera beispielsweise

Anobium punctatum,
Rhizopertha dominica,
Acanthoscelides obtectus,
Hylotrupes bajulus,
Agelastica alni,
Leptinotarsa decemlineata,
Phaedon cochleariae,
Diabrotica spp.,
Psylliodes chrysocephala,
Epilachna varivestis,
Atomaria spp.,
Oryzaephilus surinamensis,
Anthonomus spp.,
Sitophilus spp.,
Otiorrhynchus sulcatus,
Cosmopolites sordidus,
Ceuthorrhynchus assimilis,
Hypera postica,
Dermestes spp.,
Trogoderma spp.,
Anthrenus spp.,
Attagenus spp.,
Lyctus spp.,
Meligethes aeneus,
Ptinus spp.,
Niptus hololeucus,
Gibbium psylloides,
Tribolium spp.,
Tenebrio molitor,
Agriotes spp.,
Conoderus spp.,
Melolontha melolontha,
Amphimallon solstitialis und
Costelytra zealandica;

aus der Ordnung der Hymenoptera beispielsweise

Diprion spp.,
Hoplocampa spp.,
Lasius spp.,
Monomorium pharaonis und
Vespa spp.;

aus der Ordnung der Diptera beispielsweise

Aedes spp.,
Anopheles spp.,
Culex spp.,
Drosophila melanogaster,
Musca spp.,
Fannia spp.,
Calliphora erythrocephala,
Lucilia spp.,
Chrysomyia spp.,
Cuterebra spp.,
Gastrophilus spp.,

Hyppobosca spp.,
Stomoxys spp.,
Oestrus spp.,
Hypoderma spp.,
Tabanus spp.,
Tannia spp.,
Bibio hortulanus,
Oscinella frit,
Phorbia spp.,
Pegomyia hyoscyami,
Ceratitis capitata,
Dacus oleae und
Tipula paludosa.

Weiterhin können auf dem Gebiet der Veterinärmedizin die neuen Verbindungen der vorliegenden Erfindung in wirksamer Weise zur Bekämpfung einer Vielfalt schädlicher Tierparasiten (innere und äußere tierparasitische Schädlinge), z.B. parasitische Insekten und Nematoden, eingesetzt werden. Solche tierparasitischen Schädlinge können beispielhaft aus der Klasse der Insekten durch

Gastrophilus spp.,
Stomoxys spp.,
Trichodectes spp.,
Rhodnius spp.,
Ctenocephalides canis

und dergleichen verkörpert werden.

Der Begriff "Schädlingsbekämpfungsmittel", wie er hierin verwendet wird, bezieht sich auf eine Bekämpfungswirkung gegen Schädlinge, die die im Vorstehenden genannten einschließen.

Die Wirkstoffe können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der Wirkstoffe mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat

oder Latices wie Gummi arabicum, Polyvinylalkohol und Polyvinylacetat können bei der Formulierung verwendet werden.

Es ist möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% des jeweiligen Wirkstoffs.

Die erfindungsgemäßen Wirkstoffe können in ihren handelsüblichen Formulierungen oder den aus diesen Formulierungen hergestellten Anwendungsformen im Gemisch mit anderen Wirkstoffen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte, biologisch aktive Substanzen.

Die erfindungsgemäßen Wirkstoffe können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der Wirkstoffe steigern, ohne daß es für das zugesetzte synergistische Mittel erforderlich ist, selbst aktiv zu sein.

Der Gehalt des Wirkstoffs in den aus den im Handel erhältlichen Formulierungen hergestellten Anwendungsformen kann innerhalb weiter Grenzen variieren. Die Konzentration der aktiven Verbindung in den Anwendungsformen kann 0,0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die neuen Wirkstoffe durch eine hervorragende Residualwirkung auf Holz und Ton sowie eine gute Beständigkeit gegen Alkali auf gekalkten Unterlagen aus.

Die folgenden Beispiele erläutern die vorliegende Erfindung im einzelnen. Es ist jedoch ausdrücklich darauf hinzuweisen, daß die vorliegende Erfindung in keiner Weise allein auf diese Beispiele beschränkt ist.

Herstellungsbeispiele

Beispiel 1

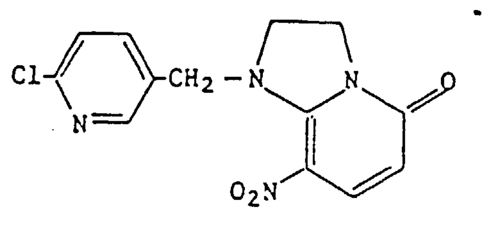

(Verbindung Nr. 2)

Ein Lösungsgemisch bestehend aus 2-Nitromethylen-3-(2-chloro-5-pyridylmethyl)imidazolidin (12,7 g), Propiolsäuremethylester (4,2 g) und Methanol (100 ml) wurde 20 h zum Rückfluß erhitzt. Anschließend wurde das Methanol unter vermindertem Druck abdestilliert, und der Rückstand wurde mittels Silicagel-Säulenchromatographie raffiniert, wonach das gewünschte 1,2,3,5-Tetrahydro-1-(2-chloro-5-pyridylmethyl)-8-nitroimidazo[1,2-a]pyridin-5-on (8 g) erhalten wurde; Schmp. 199-203 °C.

Beispiel 2

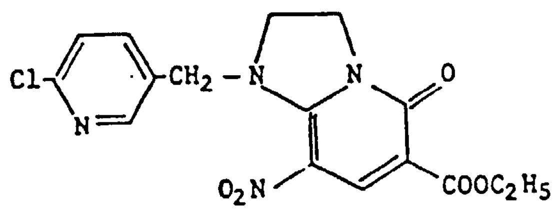

*

(Verbindung Nr. 4)

* nicht erfindungsgemäßes Beispiel zur Illustration Ethyl-1,2,3,5-Tetrahydro-8-nitro-5-oxy-imidazo[1,2-a]pyridin-6-carboxylat (12,7 g) wurde in trockenem Dimethylsulfoxid (60 ml) gelöst, 60-proz. Natriumhydrid (2 g) wurde in kleinen Anteilen in einem Strom aus Stickstoff-Gas, bei Raumtemperatur zu der Lösung hinzugefügt, und die Mischung wurde 1 h zur Gewinnung des Natrium-Salzes gerührt. Als nächstes wurde 2-Chloro-5-chloromethylpyridin (8,1 g) bei Raumtemperatur dazugegeben, und der Gefäß-Inhalt wurde einen Tag bei Raumtemperatur gerührt. Der gesamte Gefäß-Inhalt wurde vorsichtig in 100 ml eiskaltes Wasser gegossen, um die gewünschte Verbindung in Form von Kristallen abzuscheiden, die abfiltriert und mit Ethanol gewaschen wurden, wonach das gewünschte Ethyl-1,2,3,5-Tetrahydro-1-(2-chloro-5-pyridylmethyl-8-nitroimidazo[1,2-a]pyridin-5-on-6-carboxylat (12 g) erhalten wurde; Schmp. 259-263 °C.

Beispiel 3

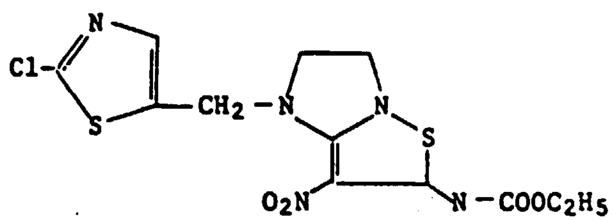

(Verbindung Nr. 63)

2-Nitromethylen-3-(2-chloro-5-thiazolylmethyl)-imidazolidin (13 g) wurde in trockenem Acetonitril (100 ml) suspendiert, Ethoxycarbonylisothiocyanat (6,6 g) wurde zu der oben genannten Suspension bei Raumtemperatur hinzugefügt, und die Mischung wurde anschließend 10 min zum Rückfluß erhitzt. Der Gefäß-Inhalt wurde auf Raumtemperatur gebracht und anschließend 1 h gerührt, um Kristalle abzuscheiden, die abfiltriert wurden.

Die Kristalle wurden als nächstes in Essigsäure (60 ml) suspendiert, wobei sie bei einer Temperatur von 10 °C gerührt wurden, und Brom (8 g) wurde tropfenweise hinzugefügt. Nach Beendigung des Zutropfens wurde die Mischung zu Vervollständigung der Reaktion 1 h weiter gerührt. Die Essigsäure wurde unter vermindertem Druck abdestilliert, Wasser wurde zu dem Rückstand zur Auflösung desselben hinzugefügt, Natriumhydrogencarbonat wurde in kleinen Anteilen zu der Mischung gegeben, um dieselbe alkalisch zu machen, und danach wurde die Mischung zweimal mit Dichloromethan extrahiert. Nachdem die Dichloromethan-Schicht mit Natriumsulfat getrocknet worden war, wurde das Lösungsmittel abdestilliert, und der Rückstand wurde aus Ethanol umkristallisiert, wonach das gewünschte Ethyl-2,4,5,6-tetrahydro-4-(2-chloro-5-thiazylmethyl)-3-nitroimidazo[1,2-b]isothiazol-2-ylidencarbamat (12 g) erhalten wurde; Schmp. 176-180 °C.

Verbindungen, die nach den gleichen Verfahren, wie sie in den vorstehenden Beispielen 1 bis 3 gezeigt wurden, hergestellt werden können, sind zusammen mit den Verbindungen der Beispiele 1 bis 3 in der folgenden Tabelle 1 aufgeführt.

## Tabelle 1

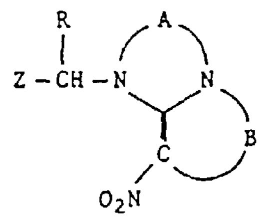

| Verbindung Nr. | Z | R | R<br>(Z-CH-N-Seite) A | (C-Seite) B (N-Seite) | |
|---|---|---|---|---|---|
| 6 | 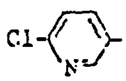 | H | $-CH_2CH_2-$ | 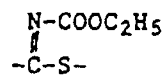 | Schmp. 131 - 135°C (Zersetzung) |

## Tabelle 1 - Fortsetzung

| Verbindung Nr. | Z | R | R<br>(Z-CH-N-Seite) A | (C-Seite) B (N-Seite) | |
|---|---|---|---|---|---|
| 8 | 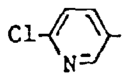 | H | $-CH_2CH_2-$ | $C(CH_3)_3$<br>$-C=CH-$ | Schmp. 148 - 150°C |
| 9 | 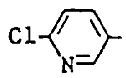 | H | $-CH_2CH_2-$ | $-CH=CH-$ | Schmp. 139 - 143°C |
| 11 | 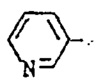 | H | $-CH_2CH_2-$ | $-CH_2CH_2-$ | |
| 12 | 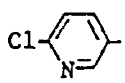 | H | $-CH_2CH_2-$ | $-CH_2CH_2-$ | |
| 13 | 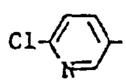 | H | $-CH_2CH_2-$ | $CH_3$<br>$-C=CH-$ | |
| 14 | 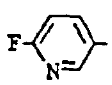 | H | $-(CH_2)_3-$ | $-CH=CH-$ | |
| 15 | 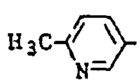 | H | $-CH_2CH_2-$ | $-CH=CH-$ | |

**Tabelle 1 - Fortsetzung**

| Verbindung Nr. | Z | R | R<br>\|<br>(Z-CH-N -Seite) A | (C-Seite) B (N-Seite) | |
|---|---|---|---|---|---|
| 16 | 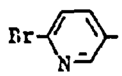 | H | $-CH_2CH_2-$ | $-CH=CH-$ | |
| 17 | 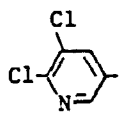 | H | $-CH_2CH_2-$ | $-CH=CH-$ | |
| 18 | 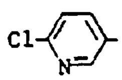 | H | $CH_3$<br>\|<br>$-CH_2CH-$ | $-CH=CH-$ | |
| 19 | 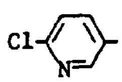 | H | $-(CH_2)_3-$ | $C(CH_3)_3$<br>\|<br>$-C=CH-$ | |
| 20 | 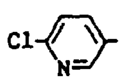 | H | $-CH_2CH_2-$ | 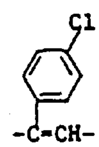 | |
| 21 | 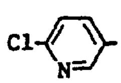 | H | $-CH_2CH_2-$ | $C(CH_3)_3$<br>\|<br>$-C=C-$<br>\|<br>Br | |
| 22 | 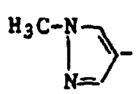 | H | $-CH_2CH_2-$ | $-CH=CH-$ | |
| 23 | 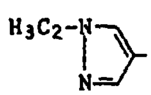 | H | $-(CH_2)_3-$ | $-CH=CH-$ | |

## Tabelle 1 - Fortsetzung

| Verbindung Nr. | Z | R | R (Z-CH-N-Seite) A | (C-Seite) B (N-Seite) | |
|---|---|---|---|---|---|
| 24 | 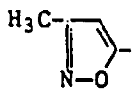 | H | $-CH_2CH_2-$ | $-CH=CH-$ | |
| 25 | 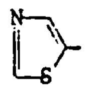 | $CH_3$ | $-CH_2CH_2-$ | $-CH_2CH_2-$ | |
| 26 | 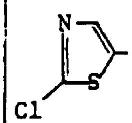 | H | $-CH_2CH_2-$ | $-CH=CH-$ | |
| 27 | 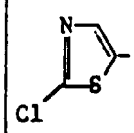 | H | $-(CH_2)_3-$ | $CH_3$ <br> $-C=CH-$ | |
| 28 | 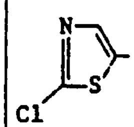 | H | $-CH_2CH_2-$ | $-CCl=CH-$ | |
| 29 | 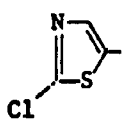 | H | $-CH_2CH_2-$ | $OCH_3$ <br> $-C=CH-$ | |
| 30 | 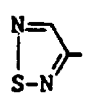 | H | $-(CH_2)_3-$ | $-CH=CH-$ | |
| 31 | 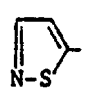 | H | $-CH_2CH_2-$ | $-CH=CH-$ | |

**Tabelle 1 - Fortsetzung**

| Verbindung Nr. | Z | R | R<br>(Z-CH-N-Seite) A | (C-Seite) B (N-Seite) | |
|---|---|---|---|---|---|
| 32 | 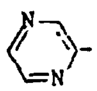 | H | $-(CH_2)_3-$ | -CH=CH- | |
| 33 | 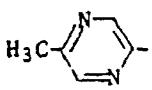 | H | $-(CH_2)_2-$ | -CH=CH- | |
| 34 | 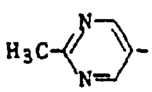 | H | $-CH_2CH_2-$ | $-CH_2CH_2-$ | |
| 35 | 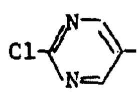 | H | $-CH_2CH_2-$ | -CH=CH- | |
| 36 | 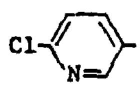 | H | $-\ CH_2CH_2-$ | $COOC_2H_5$<br>-C=CH- | |
| 37 | 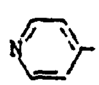 | H | $-CH_2CH_2-$ | O<br>‖<br>$-CH_2-C-$ | |
| 38 | 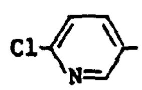 | H | $-CH_2CH_2-$ | O<br>‖<br>$-CH_2-C-$ | |
| 39 | 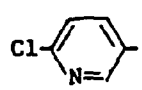 | $CH_3$ | $-(CH_2)_3-$ | O<br>‖<br>$-CH_2-C-$ | |

## Tabelle 1 - Fortsetzung

| Verbindung Nr. | Z | R | R<br>(Z-CH-N-Seite) A (C-Seite) | B (N-Seite) | |
|---|---|---|---|---|---|
| 40 | 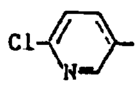 | H | $-CH_2CH_2-$ | 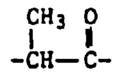 | |
| 41 | 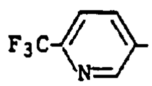 | H | $-CH_2CH_2-$ | 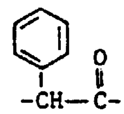 | |
| 42 | 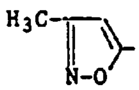 | H | $-CH_2CH_2-$ | 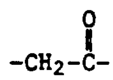 | |
| 43 | 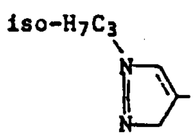 | H | $-CH_2CH_2-$ | 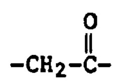 | |
| 44 | 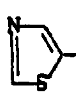 | H | $-CH_2CH_2-$ | 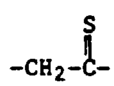 | |
| 45 | 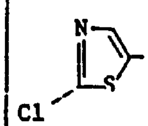 | H | $-CH_2CH_2-$ | 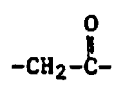 | |
| 46 | 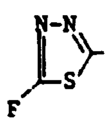 | H | 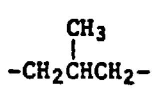 | 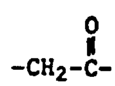 | |

**Tabelle 1 - Fortsetzung**

| Verbindung Nr. | Z | R | $(Z-\overset{R}{C}H-N$-Seite) A | (C-Seite) B (N-Seite) | |
|---|---|---|---|---|---|
| 47 | 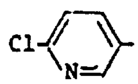 | H | $-CH_2CH_2-$ | 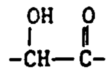 | |
| 48 | 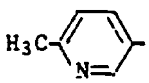 | H | $-CH_2CH_2-$ | 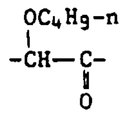 | |
| 49 | 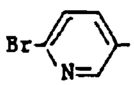 | H | $-CH_2CH_2-$ | 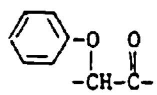 | |
| 50 | 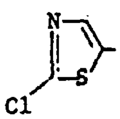 | H | $-CH_2CH_2-$ | 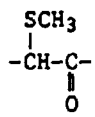 | |
| 51 | 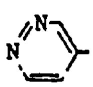 | H | $-CH_2CH_2-$ | 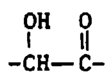 | |
| 52 | 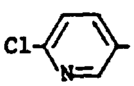 | H | $-CH_2CH_2-$ | 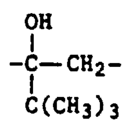 | |
| 53 | 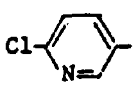 | H | $-CH_2CH_2-$ | 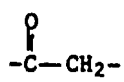 | |
| 54 | 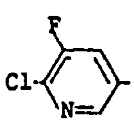 | H | $-CH_2CH_2-$ | 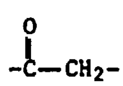 | |

**Tabelle 1 - Fortsetzung**

| Verbindung Nr. | Z | R | R<br>(Z-CH-N-Seite) A | (C-Seite) B (N-Seite) | |
|---|---|---|---|---|---|
| 55 | 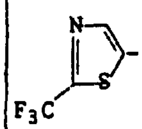 | H | $-(CH_2)_3-$ | 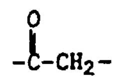 | |
| 56 | 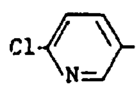 | H | $-(CH_2)_3-$ | 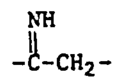 | |
| 57 | 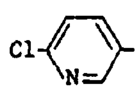 | H | $-CH_2CH_2-$ | 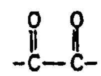 | |
| 58 | 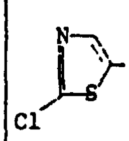 | H | $-(CH_2)_3-$ | 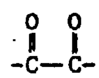 | |
| 59 | 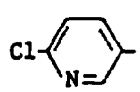 | H | $-CH_2CH_2-$ | 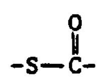 | |
| 60 | 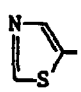 | H | $-(CH_2)_3-$ | 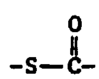 | |
| 61 | 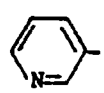 | H | $-CH_2CH_2-$ | 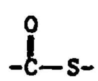 | |
| 62 | 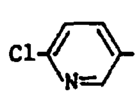 | H | $-CH_2CH_2-$ | 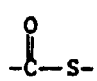 | |

Tabelle 1 - Fortsetzung

| Verbindung Nr. | Z | R | R<br>(Z-CH-N-Seite) A | (C-Seite) B (N-Seite) | |
|---|---|---|---|---|---|
| 63 | 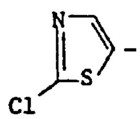 | H | $-CH_2CH_2-$ | 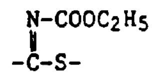 | Schmp. 176 - 180°C |
| 64 | 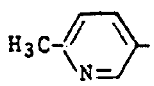 | H | $-CH_2CH_2-$ | 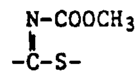 | |
| 65 | 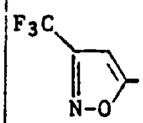 | H | $-(CH_2)_3-$ | 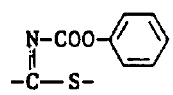 | |
| 66 | 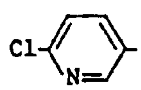 | H | $-CH_2CH_2-$ | 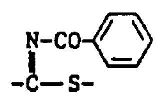 | |
| 67 | 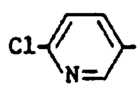 | H | $-CH_2CH_2-$ | -CH=N- | |
| 68 | 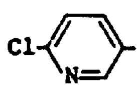 | H | $-CH_2CH_2-$ | 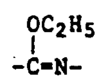 | |
| 69 | 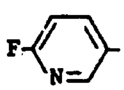 | H | $-(CH_2)_3-$ | 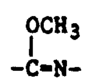 | |
| 70 | 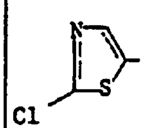 | H | $-CH_2CH_2-$ | 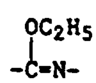 | |

Tabelle 1 - Fortsetzung

| Verbindung Nr. | Z | R | (Z-CH(R)-N-Seite) A | (C-Seite) B (N-Seite) | |
|---|---|---|---|---|---|
| 71 | 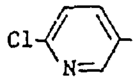 | H | $-CH_2CH_2-$ | 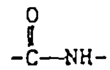 | |
| 72 | 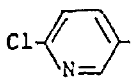 | H | $-CH_2CH_2-$ | 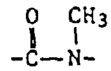 | |
| 73 | 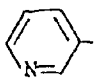 | H | $-CH_2CH_2-$ | -N=N- | |
| 74 | 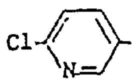 | H | $-CH_2CH_2-$ | -N=N- | |
| 75 | 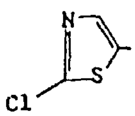 | H | $-CH_2CH_2-$ | -N=N- | |

Tabelle 1 - Fortsetzung

| Verbindung Nr. | Z | R | (Z-CH(R)-N-Seite) A | (C-Seite) B (N-Seite) | |
|---|---|---|---|---|---|
| 137 | 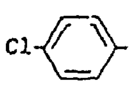 | H | $-CH_2CH_2-$ | -CH=CH- | |
| 138 | 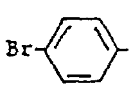 | H | $-CH_2CH_2-$ | 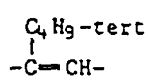 | |

## Tabelle 1 - Fortsetzung

| Verbindung Nr. | Z | R | R<br>(Z-CH-N-Seite) A | (C-Seite) B (N-Seite) | |
|---|---|---|---|---|---|
| 139 | 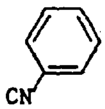 | H | $-CH_2CH_2-$ | $-CH=CH-$ | |
| 140 | 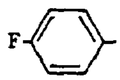 | H | $-CH_2CH_2-$ | 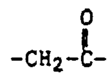 | |
| 141 | 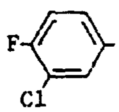 | H | $-(CH_2)_3-$ | 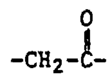 | |
| 142 | 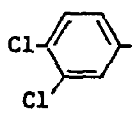 | H | $-CH_2CH_2-$ | 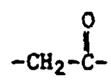 | |
| 143 | 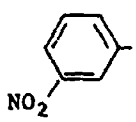 | H | $-CH_2CH_2-$ | 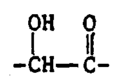 | |
| 144 | 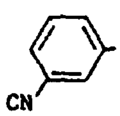 | H | $-CH_2CH_2-$ | 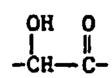 | |
| 145 | 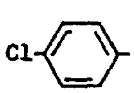 | H | $-CH_2CH_2-$ | 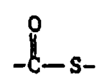 | |

**Tabelle 1 - Fortsetzung**

| Verbindung Nr. | Z | R | R<br>(Z-CH-N-Seite) A | (C-Seite) B (N-Seite) | |
|---|---|---|---|---|---|
| 146 | 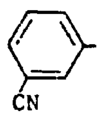 | H | $-CH_2CH_2-$ | 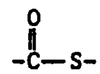 | |
| 147 | 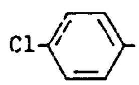 | H | $-(CH_2)_3-$ | 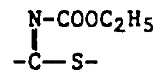 | |
| 148 | 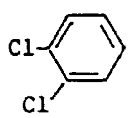 | H | $-CH_2CH_2-$ | 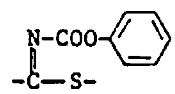 | |
| 149 | 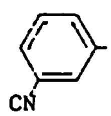 | H | $-CH_2CH_2-$ | 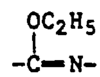 | |
| 150 | 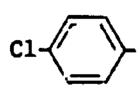 | H | $-CH_2CH_2-$ | 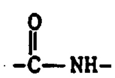 | |
| 151 | 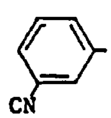 | H | $-(CH_2)_3-$ | -N=N- | |
| 152 | 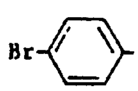 | H | $-CH_2CH_2-$ | -CH=CH- | |

Biologische Beispiele

Vergleichs-Verbindungen:

C-1:

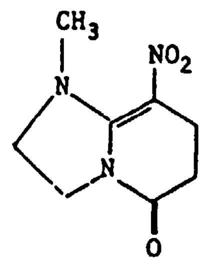

(beschrieben in Chemische Berichte, 1968, Band 119, Seiten 2208-2219)

C-2:

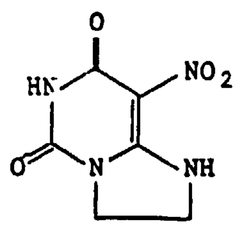

(beschrieben in Heterocycles, 1980, Band 15, Seite 437)

Beispiel 4

Test durchgeführt gegen Nephotettix cincticeps, der gegenüber Insektiziden der Organophosphor-Reihe Resistenz zeigte

Herstellung einer Test-Formulierung:

| | | |
|---|---|---|
| Lösungsmittel: | 3 Gew.-Teile | Xylol |
| Emulgator: | 1 Gew.-Teil | Polyoxyethylenalkylphenylether |

Zur Herstellung einer geeigneten Formulierung eines Wirkstoffs wurde 1 Gew.-Teil des Wirkstoffs mit der oben bezeichneten Menge Lösungsmittel, das die oben angegebene Menge Emulgator enthielt, vermischt, und die Mischung wurde mit Wasser auf die vorher festgesetzte Konzentration verdünnt.

Test-Verfahren:

Benutzt wurde eine Anzahl Töpfe mit jeweils einem Durchmesser von 12 cm, in die Reispflanzen-Setzlinge mit einer Höhe von etwa 10 cm gesetzt waren.

Auf jeden eingetopften Reispflanzen-Setzling wurden 10 ml einer wäßrigen Lösung des oben präparierten Wirkstoffs mit der vorher festgelegten Konzentration gesprüht.

Nachdem die gesprühte Lösung eingetrocknet war, wurde jeder der Töpfe mit einem Drahtkorb von 7 cm Durchmesser und 14 cm Höhe bedeckt, in dem 30 ausgewachsene weibliche Exemplare von Nephotettix cincticeps, die gegen Insektizide der Organophosphor-Reihe Resistenz zeigten, ausgesetzt wurden, und jeder Topf wurde in einen Raum mit konstanter Temperatur gestellt. Zwei Tage danach wurde die Zahl der toten Insekten ermittelt, um die Mortalitäts-Rate der Insekten zu erhalten.

Die Test-Ergebnisse wurden auf Mittelwert-Basis bestimmt und sind in Tabelle 2 aufgeführt.

Tabelle 2

| Verbindung Nr. | Wirkstoffkonzentration in ppm | Insekten-Mortalität % |
|---|---|---|
| * 1 | 200 | 100 |
| * 4 | 200 | 100 |
| 6 | 200 | 100 |
| 8 | 200 | 100 |
| 9 | 200 | 100 |
| Vergleichs-Verbindungen | | |
| C-1 | 1000 | 20 |
| C-2 | 1000 | 12 |

* nicht erfindungsgemäße Verbindungen

Beispiel 5

Test durchgeführt gegen Myzus persicae, der gegenüber Insektiziden der Organophosphor- und Carbamat-Reihe Resistenz zeigte

Test-Verfahren:

Auf Auberginen-Setzlinge (schwarze längliche Auberginen) mit jeweils einer Höhe von 20 cm, die in unglasierten Töpfen von 15 cm Durchmesser gezogen worden waren, wurden pro Setzling 200 herangezogene Exemplare von Myzus persicae der gegenüber Insektiziden der Organophosphor- und Carbamat-Reihe Resistenz besaß, ausgesetzt. Einen Tag nach der Inokulierung wurde eine wäßrige Lösung mit einer vorher festgelegten Konzentration des Wirkstoffs, der nach einer Arbeitsweise ähnlich derjenigen von Beispiel 4 beschrieben hergestellt worden war, in einer genügenden Dosierung auf die Setzlinge gesprüht.

Der oben angegebene Test wurde für jede der nachstehend bezeichneten Verbindungen mit den angegegebenen Konzentrations-Dosierungen durchgeführt. Nach dem Sprühen der insektiziden Lösung wurden die Setzlinge für den Test in jedem Topf 24 h in einem auf einer Temperatur von 28 °C gehaltenen Gewächshaus stehen gelassen, und danach wurde die Mortalitäts-Rate der Insekten für jeden Test bestimmt. Der gleiche Test wurde zweimal wiederholt, um eine genaue Bestimmung der Mortalitäts-Rate zu erhalten. Das Ergebnis ist in der nachfolgenden Tabelle 3 dargestellt:

Tabelle 3

| Verbindung Nr. | Wirkstoffkonzentration in ppm | Insekten-Mortalität % |
|---|---|---|
| * 1 | 200 | 100 |
| * 4 | 200 | 100 |
| 6 | 200 | 100 |
| 8 | 200 | 100 |
| 9 | 200 | 100 |
| Vergleichs-Verbindungen | | |
| C-1 | 1000 | 0 |
| C-2 | 1000 | 0 |

* nicht erfindungsgemäße Verbindungen

**Patentansprüche**

1. Nitro-substituierte heterocyclische Verbindungen der Formel (I)

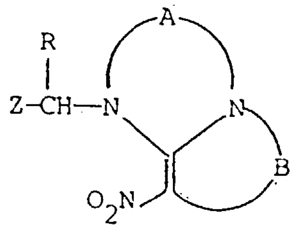

(I)

in der

R Wasserstoff oder $C_1$-$C_4$-Alkyl ist,
Z $C_6$-$C_{10}$-Aryl oder eine 5- bis 6-gliedrige heterocyclische Gruppe, die 1 bis 2 aus O, S und N ausgewählte Hetero-Atom enthält, von denen wenigstens eines ein Stickstoff-Atom ist, ist, wobei die Aryl-Gruppe oder die heterocyclische Gruppe jeweils durch einen Substituenten substituiert sein kann, der beliebig aus der aus Halogen, $C_1$-$C_4$-Alkyl, $C_1$-$C_4$-Alkoxy, $C_1$-$C_4$-Halogenoalkyl, $C_1$-$C_4$-Alkylthio, $C_1$-$C_4$-Halogenoalkoxy, $C_1$-$C_4$-Halogenoalkylthio, Nitro und Cyano bestehenden Gruppe ausgewählt ist,
A Ethylen oder Trimethylen ist, die beide durch Methyl substituiert sein können, und
B für 2 Glieder eines heterocyclischen Ringes steht, der zusammen mit dem benachbarten C-Atom und dem benachbarten N-Atom gebildet wird und worin wenigstens eines der Glieder, die gegebenenfalls substituiert sein können, ein Stickstoff-Atom oder ein Schwefel-Atom bezeichen kann,

wobei die Glieder B gegebenenfalls durch wenigstens einen Substituenten substituiert sein können, der aus der aus Halogen, $C_1$-$C_6$-Alkyl, $C_1$-$C_4$-Alkoxy, Alkoxycarbonyl mit 1 bis 4 Kohlenstoff-Atomen in dem Alkoxy-Teil, $C_6$-$C_{10}$-Aryl, Keto, Imino, Phenoxy, $C_1$-$C_4$-Alkoxythio, Alkoxycarbonylimino mit 1 bis 4 Kohlenstoff-Atomen in dem Alkoxy-Teil, Phenoxycarbonylimino, Benzoylimino, Benzyl, Cyano, Thioketo, Hydroxy und $C_1$-$C_2$-Alkyliden bestehenden Gruppe ausgewählt ist.

**2.** Nitrosubstituierte heterocyclische verbindungen der Formel

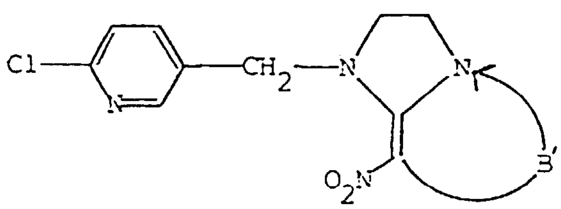

worin
B' für eine der folgenden Gruppierungen steht:

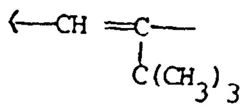

,

$\leftarrow CH = CH—$ ,

**3.** Verfahren zur Herstellung von nitro-substituierten heterocyclischen Verbindungen der Formel (I) gemäß Anspruch 1

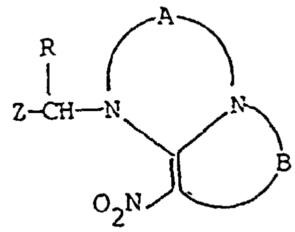

(I)

in der R, Z, A und B die im Anspruch 1 angegebene Bedeutung haben,
dadurch gekennzeichnet, daß
Verbindungen der Formel (II)

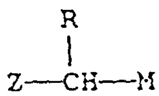

(II),

in der

R und Z jeweils die im Vorstehenden angegebenen Bedeutungen haben,
M Halogen oder $-OSO_2-L$, worin L Alkyl oder Aryl bezeichnet, das substituiert sein kann, darstellt,

mit Verbindungen der Formel (III)

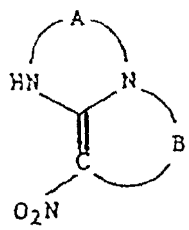

(III)

,

in der A und B die im Vorstehenden angegebenen Bedeutungen haben, in Gegenwart inerter Lösungsmittel und gegebenenfalls in Gegenwart einer Base umgesetzt werden.

**4.** Insektizide Mittel, dadurch gekennzeichnet, daß sie wenigstens eine nitro-substituierte heterocyclische Verbindung der Formel (I) gemäß Anspruch 1 enthalten.

**5.** Verwendung nitro-substituierter heterocyclischer Verbindungen der Formel (I) gemäß Anspruch 1 zur Bekämpfung von Schadinsekten.

**6.** Verfahren zur Herstellung insektizider Mittel, dadurch gekennzeichnet, daß nitro-substituierte heterocyclische Verbindungen der Formel (I) gemäß Anspruch 1 mit Streckmitteln und/oder grenzflächenaktiven Mitteln vermischt werden.

## Claims

**1.** Nitro-substituted heterocyclic compounds of the formula (I)

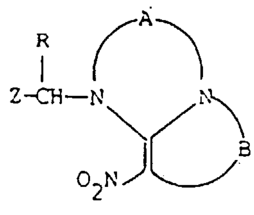

(I)

in which

- R represents hydrogen or $C_1$-$C_4$-alkyl,
- Z represents $C_6$-$C_{10}$-aryl or a 5- to 6-membered heterocyclic group containing 1 to 2 heteroatoms selected from the group consisting of O, S and N, of which at least one is a nitrogen atom, where the aryl group or the heterocyclic group may in each case be substituted by any substituent selected from the group consisting of halogen, $C_1$-$C_4$-alkyl, $C_1$-$C_4$-alkoxy, $C_1$-$C_4$-halogenoalkyl, $C_1$-$C_4$-alkylthio, $C_1$-$C_4$-halogenoalkoxy, $C_1$-$C_4$-halogenoalkylthio, nitro and cyano,
- A represents ethylene or trimethylene, each of which may be substituted by methyl, and
- B represents 2 members of a heterocyclic ring which is formed together with the neighbouring C-atom and the neighbouring N-atom and in which at least one of the members, which may optionally be substituted, denotes a nitrogen atom or a sulphur atom,

the members B being optionally substituted by at least one substituent selected from the group consisting of halogen, $C_1$-$C_6$-alkyl, $C_1$-$C_4$-alkoxy, alkoxycarbonyl having 1 to 4 carbon atoms in the alkoxy moiety, $C_6$-$C_{10}$-aryl, keto, imino, phenoxy, $C_1$-$C_4$-alkoxythio, alkoxycarbonylimino having 1 to 4 carbon atoms in the alkoxy moiety, phenoxycarbonylimino, benzoylimino, benzyl, cyano, thioketo, hydroxyl and $C_1$-$C_2$-alkylidene.

**2.** Nitro-substituted heterocyclic compounds of the formula

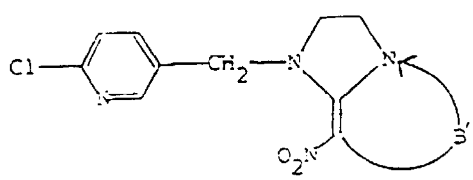

in which
B' represents one of the following groups:

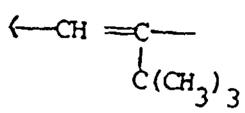

,

$$\leftarrow CH = CH— ,$$

**3.** Process for preparing nitro-substituted heterocyclic compounds of the formula (I) according to Claim 1

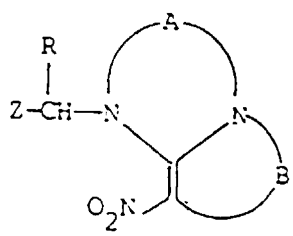

(I)

in which R, Z, A and B are each as defined in Claim 1,
characterized in that
compounds of the formula (II)

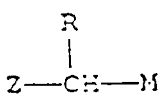

(II),

in which

R and Z are each as defined above,
M represents halogen or $-OSO_2-L$ in which L denotes alkyl or aryl with or without substitution,

are reacted with compounds of the formula (III)

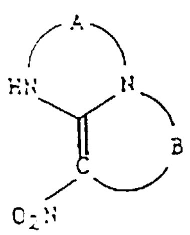

(III)

in which A and B are each as defined above, in the presence of inert solvents and optionally in the presence of a base.

**4.** Insecticides characterized in that they comprise at least one nitro-substituted heterocyclic compound of the formula (I) according to Claim 1.

**5.** Use of nitro-substituted heterocyclic compounds of the formula (I) according to Claim 1 for controlling harmful insects.

**6.** Process for preparing insecticidal compositions, characterized in that nitro-substituted heterocyclic compounds of the formula (I) according to Claim 1 are mixed with extenders and/or surface-active agents.

**Revendications**

**1.** Composés hétérocycliques substitués par un groupe nitro, répondant à la formule (I)

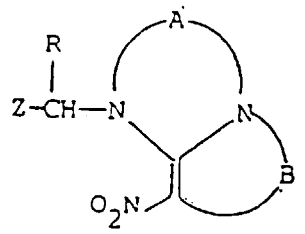

(I)

dans laquelle

R représente un atome d'hydrogène ou un groupe alkyle en $C_1$-$C_4$,

Z représente un groupe aryle en $C_6$-$C_{10}$ ou encore un groupe hétérocyclique penta- à hexagonal qui contient de 1 à 2 hétéroatomes choisis parmi O, S et N, au moins un d'entre eux représentant un atome d'azote, dans lequel le groupe aryle ou le groupe hétérocyclique peut être respectivement substitué par un substituant qui peut être sélectionné librement parmi le groupe constitué par un atome d'halogène, un groupe alkyle en $C_1$-$C_4$, un groupe alcoxy en $C_1$-$C_4$, un groupe halogénalkyle en $C_1$-$C_4$, un groupe alkyl(en $C_1$-$C_4$)thio, un groupe halogénalcoxy en $C_1$-$C_4$, un groupe halogénalkyl(en $C_1$-$C_4$)thio, un groupe nitro et un groupe cyano,

A représente un groupe éthylène ou un groupe triméthylène, les deux pouvant être substitués par un groupe méthyle, et

B représente 2 chaînons d'un noyau hétérocyclique qui est formé conjointement avec l'atome C voisin et l'atome N voisin, et dans lequel au moins un des chaînons, qui peut être éventuellement substitué, peut représenter un atome d'azote ou un atome de soufre,

dans lequel les chaînons B peuvent porter, le cas échéant, au moins un substituant qui est sélectionné parmi le groupe constitué par un atome d'halogène, un groupe alkyle en $C_1$-$C_6$, un groupe alcoxy en $C_1$-$C_4$, un groupe alcoxycarbonyle contenant de 1 à 4 atomes de carbone dans la fraction alcoxy, un groupe aryle en $C_6$-$C_{10}$, un groupe céto, un groupe imino, un groupe phénoxy, un groupe alcoxy(en $C_1$-$C_4$)thio, un groupe alcoxycarbonylimino contenant de 1 à 4 atomes de carbone dans la fraction alcoxy, un groupe phénoxycarbonylimino, un groupe benzoylimino, un groupe benzyle, un groupe cyano, un groupe thiocéto, un groupe hydroxyle et un groupe alkylidène en $C_1$-$C_2$,

2. Composés hétérocycliques substitués par un groupe nitro, répondant à la formule

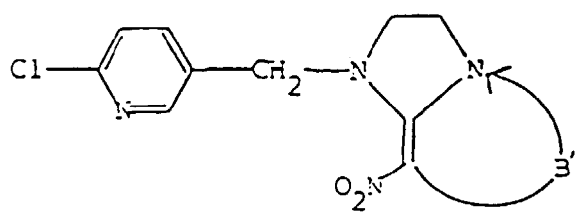

dans laquelle
B' représente un des groupements ci-après:

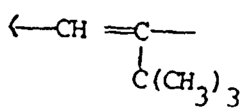

,

←CH=CH— ,

3. Procédé pour la préparation de composés hétérocycliques substitués par un groupe nitro, répondant à la formule

(I) selon la revendication 1

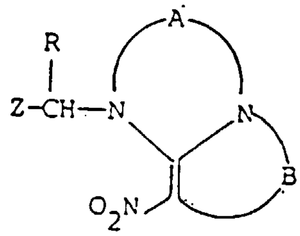

(I)

dans laquelle R, Z, A et B ont la signification indiquée à la revendication 1, caractérisé en ce qu'on fait réagir des composés de formule (II)

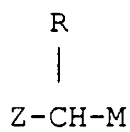

(II)

dans laquelle

R et Z ont respectivement les significations indiquées ci-dessus,
M représente un atome d'halogène ou $-OSO_2-L$ où L représente un groupe alkyle ou un groupe aryle qui peut être substitué,

avec des composés de formule (III)

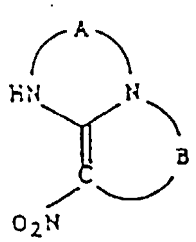

(III)

dans laquelle A et B ont les significations indiquées ci-dessus, en présence de solvants inertes et éventuellement en présence d'une base.

**4.** Agents insecticides caractérisés en ce qu'ils contiennent au moins un composé hétérocyclique substitué par un groupe nitro, répondant à la formule (I) selon la revendication 1.

**5.** Utilisation de composés hétérocycliques substitués par un groupe nitro, répondant à la formule (I) selon la revendication 1, pour lutter contre des insectes nuisibles.

**6.** Procédé pour la préparation d'agents insecticides, caractérisé en ce qu'on mélange des composés hétérocycliques substitués par un groupe nitro, répondant à la formule (I) selon la revendication 1, avec des diluants et/ou des agents tensioactifs.